# EUROPEAN PATENT APPLICATION

(11) **EP 3 777 649 A1**
(43) Date of publication of application: **17.02.2021**
(21) Application number: 20190548.6
(22) Date of filing: 11.08.2020
(51) Int. Cl.: A61B 1/247, A61B 17/02, A61B 90/00

(54) **DENTAL RETRACTOR WITH MICRO MIRROR**

(30) Priority: 14.08.2019 US 201916540929
(71) Applicant: B&L Biotech, Inc., Ansan-si, Gyeonggi-do 15462 (KR)
(72) Inventor: LEE, In Whan, 15462 Ansan-si, Gyeonggi-do (KR)
(74) Representative: Klunker IP Patentanwälte PartG mbB

(57) **Abstract**

This invention is related to a dental retractor including a body part having a handle portion, a neck portion extending from the handle portion, and a mirror part having a micro mirror, which is detachably attached to an end of the neck portion, such that the projection can be inserted in to a hole formed at a surgical site to observe an inside the hole. According to the present invention, since the dental retractor has the mirror part for observing the interior of the oral cavity, the number of operations, the number of persons required for the operation and the working time are reduced and it is not necessary to separately use a dental mirror. In addition, since the dental retractor has a mirror such as a micro mirror at the head portion, a doctor or other practitioner/ technician can use the other hand to perform a medical examination.

## Description

### Field of Invention

The present invention relates to a dental retractor capable of retraction and observing an inside of a mouth with the retractor and observing the inside of the hole or incised area using a micro mirror during a surgery.

### Background for the Invention

In general, procedures such as implant and orthodontic treatment require the gums to be cut (to make a hole) since the doctor must treat the patient with a variety of treatment instruments that are inserted between the cut of the gums. Thus, it is necessary to keep the incised gums open as much as possible.

When the patient's mouth is opened, and the patient's cheeks are not properly gripped when cutting the gum tissue covering the teeth to treat the root of teeth, it will cause a significant disruption to the treatment. For this reason, the retractor is used to secure the visual field in the patient's mouth during various dental visits or operations and to fix the gingival tissue to cover the teeth for smooth operation.

Additionally, teeth located inside the oral cavity such as molars are difficult to discern, or access, and it is necessary to support and secure the area of the mouth with a retractor for proper visual field within the oral cavity.

With the field of view in the oral cavity using the retractor, the dental mirror is inserted into the oral cavity to examine the area to be treated and appropriate treatment is performed according to the condition of the treatment area. In the past, a retractor was used to secure a view in the oral cavity, and a separate micro mirror was inserted.

However, this method requires an operation of securing the retractor, and inserting the micro mirror separately into the mouth. This increases the number of persons required for the operation and the working time since the retractor and the mirror must be separately used.

In addition, in case of a surgery, inserting or entering the entrance of the mirror into the inside of the hole is difficult, the doctor may be unable see the inside of the hole and suffer from its inconvenience, and making surgery and treatment more difficult.

Some patent literature (e.g., US 5,882,195, US 8,651,862, and US 9,610,009) disclose retractors having a mirror. However, the retractor of US 9,610,009 is designed to be fixed between a gum and a cheek of a patient, and thus a doctor cannot easily change the mirror's angle to secure a view of the treatment site. Although US 5,882,195 and US 8,651,862 disclose retractors each having a mirror, the mirrors are a regular sized mirror, which is too large to observe inside of a hole. Consequently, even in the inventions of US 5,882,195 and US 8,651,862, a micro mirror must be inserted, in addition to a retractor and a tool, into the oral cavity to observe inside of a hole.

In order to resolve such problems, the inventors made intensive studies and invented the present invention.

### Summary of the Invention

The present invention provides a dental retractor capable of retraction and observing the inside of a mouth with only one retractor and observing the inside of the hole using a micro mirror during a surgery.

In order to solve the issues as discussed above, the present invention provides a dental retractor for securing a visual field in a patient's oral cavity during a dental treatment or surgery, or for securing the incised portion of the gum, to keep the incised area (hole) exposed. This retractor includes a handle portion, a head portion, and a mirror portion. The head part and the mirror part are integrated and the mirror part can be replaced according to the use or treatment.

The head portion is disposed on one side of the handle portion and in contact with the skin tissue inside the oral cavity and has a mirror (e.g., a micro mirror), at least one side of which is a reflective mirror and can secure a field of view to a hole in a surgical site.

According to the present invention, since the dental retractor includes the mirror portion at the head portion for observing the interior of the oral cavity such as a hole formed in the tooth root, the number of operations, the number of persons required for the operation, and the working time can be reduced, and a dental mirror is not separately required. In addition, since the head portion is formed as a mirror in the dental retractor, the other hand is available to perform other tasks such as a further medical examination.

### Description of Drawings

Fig. 1A shows a dental retractor of an embodiment of the present invention (a front view and a back view).
Fig. 1B shows a dental retractor of an embodiment of the present invention (a top view and a bottom view).
Fig. 1C shows a dental retractor of an embodiment of the present invention (perspective views).
Fig. 1D shows embodiments of the present invention (left and right side views).
Fig. 1E shows embodiments of the present invention with dimensions and an angle of a neck portion.
Fig. 2 shows another embodiment of a dental retractor with mirror parts, which can be replaced.

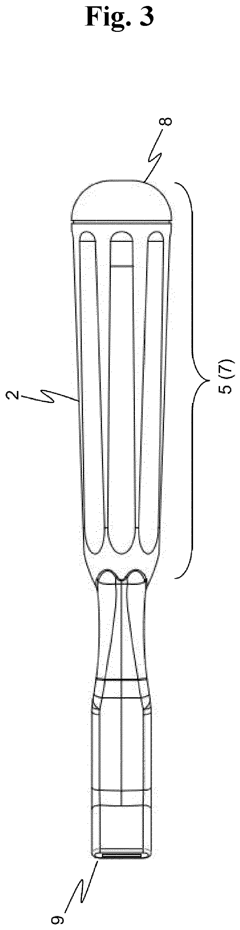

Fig. 3 shows an embodiment of a body part (having a wider neck portion) (a top view).

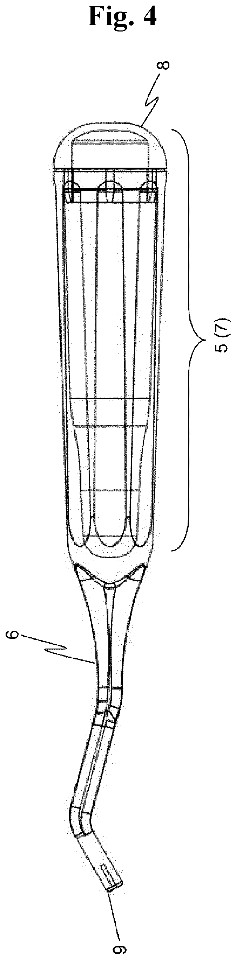

Fig. 4 is a side view of the embodiment of Fig.3.

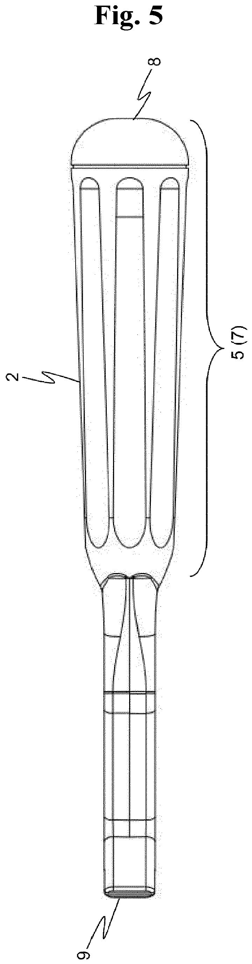

Fig. 5 shows an embodiment of a body part (having a narrower neck portion) (a top view).

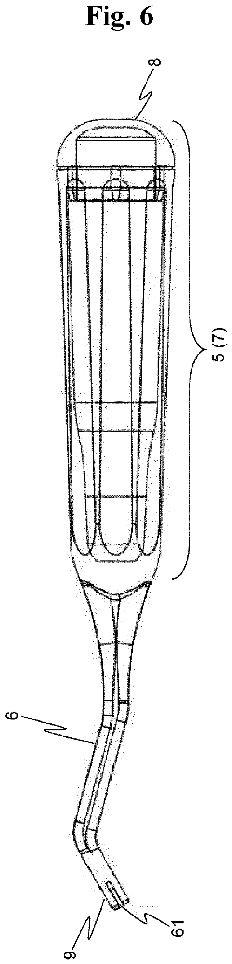

Fig. 6 is a side view of the embodiment of Fig. 5.
Fig 7 shows examples of mirror parts each having a blade-shape.
Fig. 8 shows examples of mirror parts each having a blade-shape with a micro mirror.
Fig. 9 shows an example of surgery by using the dental retractor with a protector.
Fig. 10 shows an example of surgery by using the dental retractor with a micro mirror (wide version).
Fig. 11 shows an example of surgery by using the dental retractor with a micro mirror (narrow version)

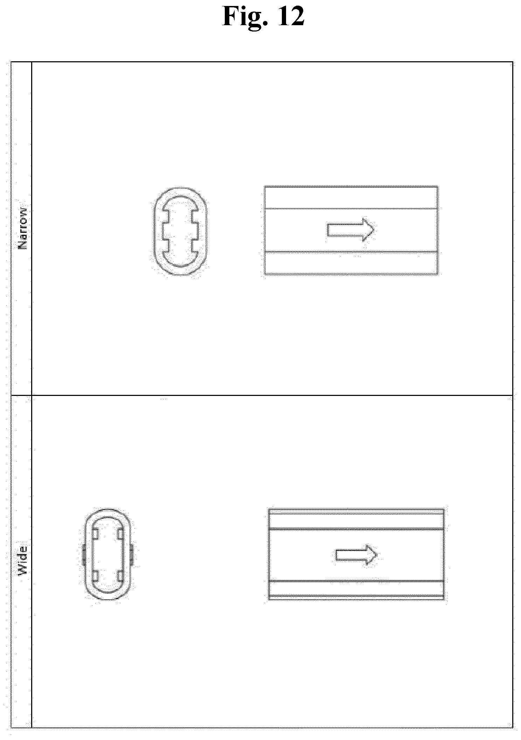

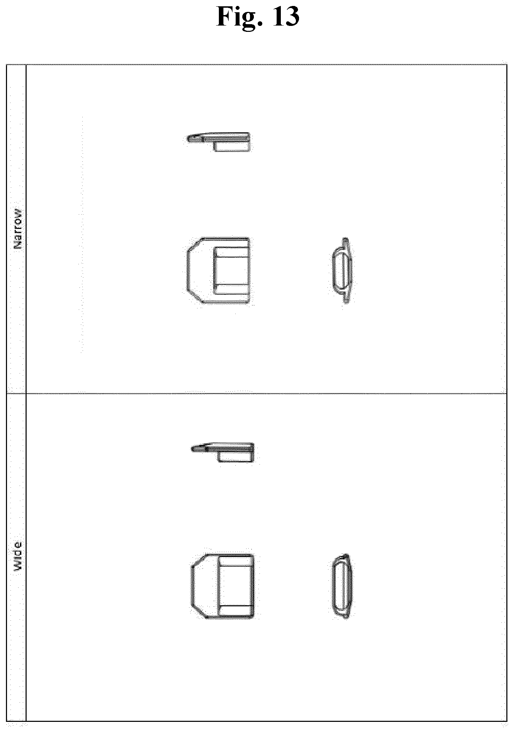

Figs. 12 and 13 show wings and autoclave caps.

### Description of Preferred Embodiments

The dental retractor of the present invention is suitable for securing a visual field in a patient's oral cavity during a dental treatment or surgery while maintaining the incised portion of the gum, which covers the teeth, in an opened state. The retractor (1) has a handle portion (5) and a head portion (3), which is located at the other side of the handle portion. More specifically, the retractor (1) is formed of a body part (2) and a mirror portion (4). A mirror portion (4) is disposed at the head portion (3) of the retractor (1). The mirror part (4) is fixed to the body part and can be replaced in accordance with the needed treatment and surgery.

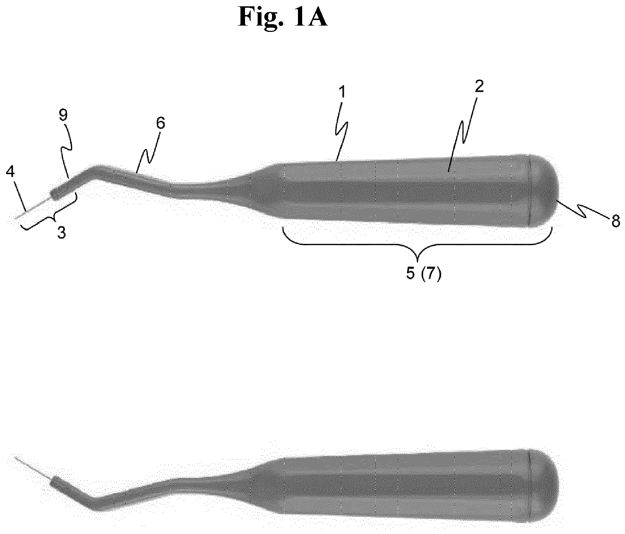

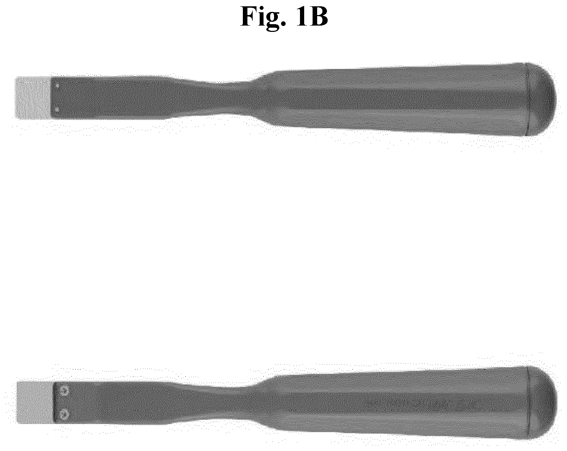

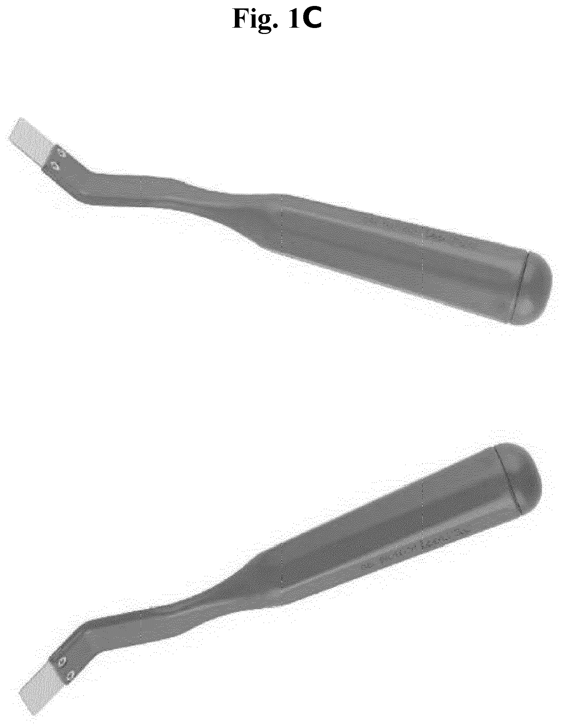

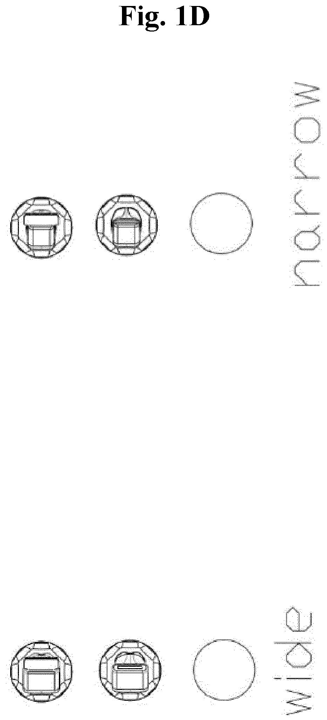

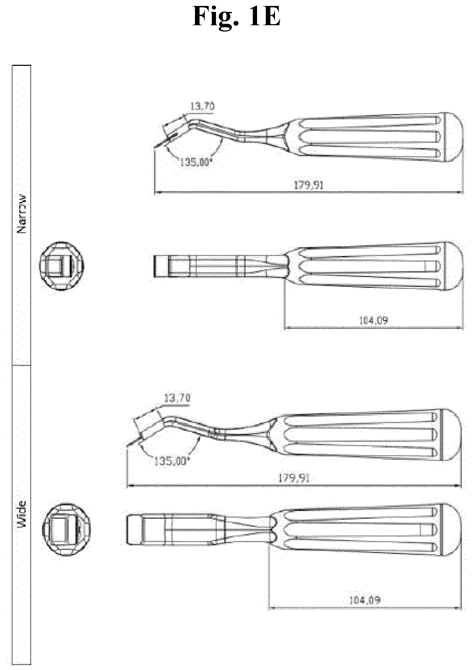

Further, the body part (2) has a handle portion (5), a neck portion (6) and a connecting portion (9) (as a part of the head portion (3)), to which a mirror part (4) can be attached. The handle portion (5) has a grip portion (7), which is formed so as to be graspable by a doctor, dentist, or technician. The head portion (3) including the mirror part (4) is located at an opposite side of the handle portion (5) so that head portion comes in contact with the skin tissue inside the oral cavity during operation (as illustrated in Figs. 1 and 10, 11).

The handle portion (5) may be tapered in a longitudinal direction from the end portion (8) toward the head portion (3), so that a doctor can securely hold the handle portion (5). Further, the handle (5) can have a circular shape, an oval shape, or polygonal shape in cross-sectional, so that the handle can be securely hold by a doctor. For example, a hexagonal cross-section is one embodiment. Further, a groove(s) may be provided at a handle to prevent slipping.

There are no limitations imposed on the material(s) of the body part. For example, the body part can be formed of a resin material, metallic material, or ceramic material or a combination thereof. Thermo-setting resin, aluminum or stainless steel or a combination thereof is preferably used for a handle material. It is preferable that body part is made from a material that is fully autoclavable for sterilization purposes.

Diameter of the grip portion is not limited but preferably 80 mm to **120** mm, more preferably **90** mm to **110** mm. The length from the end of the handle portion (8) to the end of the head portion (3) is preferably **150** mm to **200** mm, more preferably **165** mm to **185**mm.

The body part has a neck portion (6) between the handle portion (5) and the head portion (3). In general, the dimeter or width of the neck portion (6) is usually smaller that of the handle portion (5) so that a doctor can easily turn around the retractor in the mouse. The neck portion (6) can be angled or curved so that the head portion (3) can effectively reach a surgical site (e.g., gum or tooth to be treated). For example, the neck portion (6) can be bended at angle of 120 to 150 °, preferably 130 to 140 °.

A mirror part (4) can be fixed to the end or vicinity thereof of the neck portion (6) (i.e., a connecting portion (9)).

The handle portion (5) and the neck portion (6) can be formed into a one-piece structure. Alternatively, the handle portion (5) and the neck portion (6) can be separately formed and connected to each other to form a body part (i.e., a two-piece structure).

Shapes of the body part including the handle and the neck portion are not limited to the embodiments as illustrated in the Figures. It is understood that other shapes are possible keeping in spirit of the objectives described herein.

The mirror part (4) is disposed at the head portion (3) of the retractor (1). The mirror part (4) can be permanently or detachably fixed to the connecting portion (9) of the body part (2). Any conventional method can be employed to fix the mirror part (4) to the body part (2). For example, a screw(s) (21) can be used. Further, a mirror and/or the end portion of the neck can be designed to have a structure (e.g., projection or concaved portions) so that the mirror and/or the connecting portion are engaged to be fixed each other. If necessary, a glue or other adhesive also may be used.

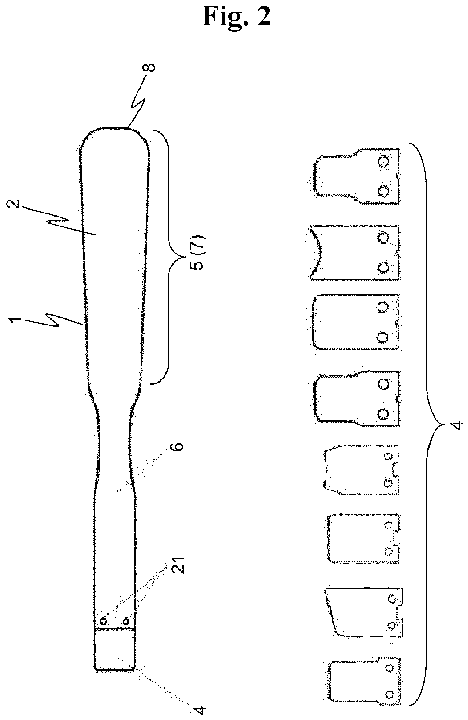

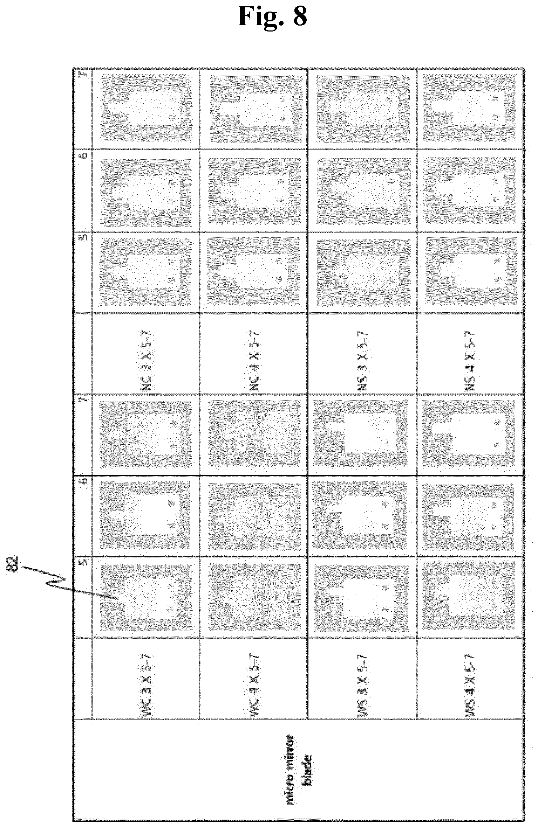

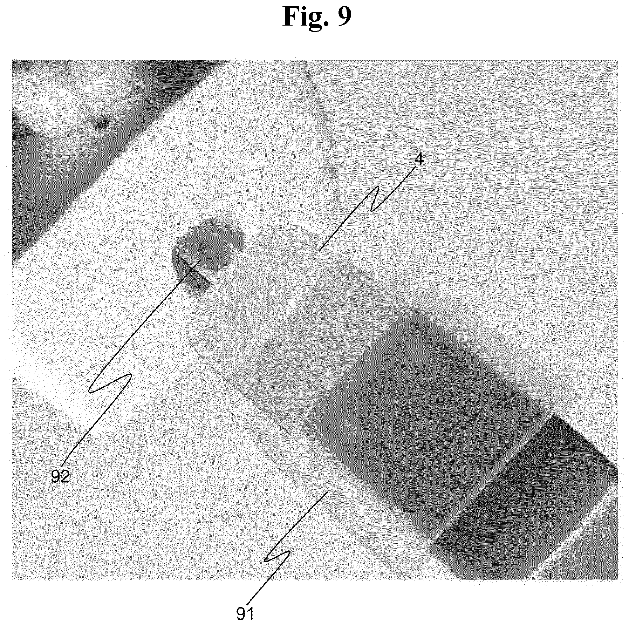

There is no limitation imposed on the shape of the mirror. For example, the shape of the mirror part can be selected from a variety of shapes such as a relatively simple shaped plate (e.g., blade), a plate shape having a projection (e.g., a blade with a micro mirror (82, 92)) in a plain view, as shown in Figs. 2 and 7-9. Since such a mirror with a micro mirror has a relatively broad portion and a relatively narrow projection (i.e., micro mirror), a doctor can observe a relatively broad area in the oral cavity by the relatively broad mirror and the inside of a hole by the micro mirror portion (see Figs. 9-11). Shapes of the mirror parts are not limited to the embodiments as illustrated in the Figures. It is understood that other shapes are possible keeping in spirit of the objectives described herein. For example, a micro mirror portion can be located at a center position of the mirror blade or an off-centered position, as shown in Fig. 8. A micro mirror portion may have a width of 3 to 4 mm and a length of 5-7 mm. Please note that in Fig. 8, "*WC*" represents embodiments having a wider mirror (main) blade with a micro mirror portion located at a center position. "*WS*" represents embodiments having a wider (main) mirror blade with a micro mirror portion located at an off-center position. "*NC*" represents embodiments having a narrower (main) mirror blade with a micro mirror located at a center position. "*NS*" represents embodiments having a narrower (main) mirror blade with a micro mirror portion located at an off-center position. Further, for example, the number "*3x5*" means that the micro mirror potion has a width of 3 mm and a length of 5 mm. Thus, for example, "*WC 3x5-7"* in Fig. 8 means that the (3) embodiments each have a wider (main) mirror blade with a micro mirror portion located at a center position, and the micro mirror portions have a width of 3 mm and a length of 5 mm, a width of 3 mm and a length of 6 mm, and a width of 3 mm and a length of 7 mm, respectively.

Further, such a relatively narrower portion of a mirror part can be used as a micro mirror and a tool to tread a narrow portion of a surgical portion (e.g., a hole) while observing the inside of a hole.

The mirror part (4) has reflective surface (mirror surface) on at least one surface, preferably the both surfaces, so that a doctor can observe the part of the oral cavity (e.g., gum and teeth) to be treated. Thus, in accordance with the dental retractor, a practitioner is capable of retraction and observing the inside of a mouth, and observing and treating the inside of the hole using the micro mirror during a surgery by using a single retractor.

It is preferable that a mirror part (4) is fixed to the body part (2) so that both surfaces of the mirror part function as a mirror. For example, as shown in Figs. 1 and 9-11, the mirror part (4) can be fixed to the end (9) of the neck so that the mirror part only partially overlaps with the connecting portion (9) of the neck portion (4). Especially, it is preferred that the mirror part is arranged so that the micro mirror portion can be inserted in a hole of surgical site (see Figs. 9-11).

The mirror part (4) can be chosen from different kinds of embodiments (e.g., see Figs. 2, 7, and 8), depending on how to treat the surgical site of a patient, since the mirror part is detachable in the present invention. The detachable mirrors, for example, have a variety of shapes, as shown in Figs. 2, 7 and 8. In the present invention, a variety of the mirrors can be used in combination with a body part depending on the situation when the retractor is arranged so that the mirror part (4) is detachably fixed thereto.

The material to form the mirror part (4) is not limited as long as the part can function as a mirror in use. For example, a stainless steel and other material (e.g., resin, metal, and ceramic) plated with silver can be preferably used. Further, it is preferable that the mirror is made from a material that is fully autoclavable for sterilization purposes.

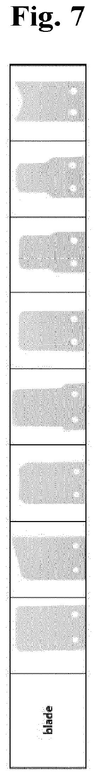

Furhermore, the mirror part may be formed to have a sharp edge. That is, in such an embodiment, the mirror serrations help to be supported when in contact with a bone structure, such as a tooth. The shape of the mirror is not limited. A simple shaped plate mirror is used to secure a view of the patient's mouth or to keep the incision open and prevent gingiva covering the teeth. The mirror has a plate shape and a protruding part, which is narrower than the main body that can be inserted into the hole from the incision at the surgical site allowing to observe the inside of the hole. See, e.g., Figures 7-11.

The connecting portion (9) of the neck portion (6) has a shape or structure so that the mirror part (4) can be fixed thereto. The shape or structure of the connecting portion (9) are not limited but preferably are formed in accordance with a shape of the mirror parts (4). For example, when a mirror part has a flat surface, it is preferable that the connecting portion (9) of the neck portion (6) has a plat surface so that the mirror part can firmly contact with the end portion of the neck. The connecting portion (9) may have a slit (61), to which a mirror part can be inserted.

For example, the mirror part (4) can be fixed to the neck portion by a screw or screws (21). When using a screw(s), the connecting portion (9) and the mirror part (4) have, respectively, at least one screw hole so that the mirror is detachably connected to the neck portion by the at least one screw.

Further, additional parts can be employed in combination with the body part (2) and the mirror part (4). For example, a protector (91) can be attached to the retractor (1). More specifically, the protector (91) can be attached to the retractor (1) so as to fully or partially cover the mirror part (4) (as shown in Fig. 9) so as to prevent the mirror part (4) directly contacting an inner wall of a cheek of a patient. A method to attach such a protector to the retractor is not limited. For example, a protector can be formed to have a thin rectangular hole (like a sheath), into which the mirror blade can be slidably inserted. There is no limitation on materials to form such a protector. For example, a resin material (e.g., thermoplastic resin or thermosetting resin), a metallic material, and/or a ceramic material can be used for such a protector. In addition, any additional parts may be attached to the retractor as long as a purpose of the retractor is not substantially impaired. The material used can also be possible to be autoclaved or sterilized.

*"Autoclaving cap"* and *"wings"* can be also employed in combination with the retractor. For example, a wing can be disposed on a mirror blade to prevent the inconvenience or injury by touching the mouth and to secure space in the mouth. An autoclaving cap can be used to cover the blade when autoclaving to prevent being scratched. The material for caps and wings can also be possible to be autoclaved or sterilized.

According to the present invention, since the dental retractor has the mirror part for observing the interior of the oral cavity, the number of operations, the number of persons required for the operation and/or the working time are reduced and it is not necessary to separately use a dental mirror.

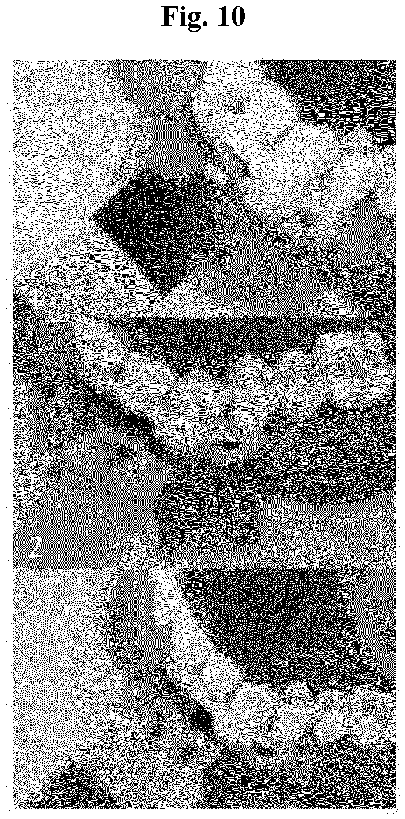

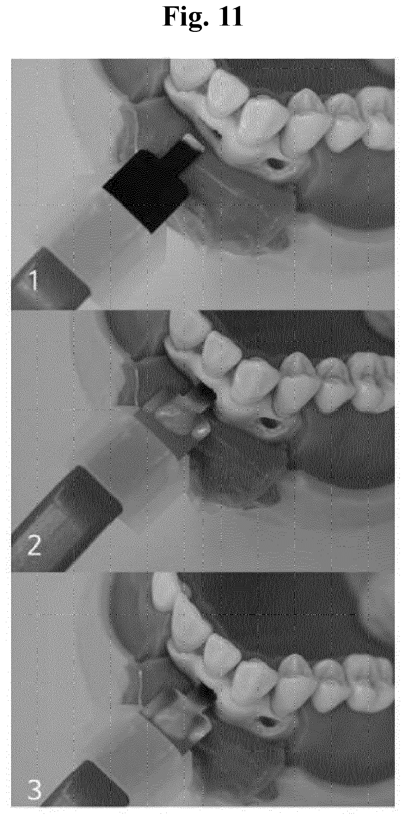

An oral cavity can be treated by the retractor of the present invention. For example, as shown in Figs. 10 and 11, after incising a skin tissue (e.g., gum), a doctor can hold the skin tissue with the retractor, which has been incised, away from a tooth or a tooth root so as to secure a vision field, then observe the inside of the hole with a micro mirror (92).

As described above, according to the present invention, since the dental retractor has the mirror part for observing the interior of the oral cavity, the number of operations, the number of persons required for the operation and/or the working time are reduced and it is not necessary to separately use a dental mirror. In addition, since the dental retractor has a mirror (e.g., a micro mirror) at the head portion, a doctor or other practitioner/ technician can use the other hand to perform a medical examination.

Further, since the mirror portion is formed to be vertically adjustable in its angle, it is possible to secure a field of view in a desired direction by adjusting the angle of the mirror part or the head portion. Further, by using a micro mirror, it is possible to observe the inside of the hole of a tooth or a tooth root during surgery, and not change tools for surgery or treatment.

Although the above description contains many specificities, these should not be construed as limiting the scope of the invention but as merely providing illustrations of some of the presently preferred embodiments of the present invention. It is to be understood that the present invention is not limited to the embodiments described above, but encompasses any and all embodiments within the scope of the claims so long as the objectives of the invention are met.

## Claims

1. A dental retractor comprising:
a body part having a handle portion and a neck portion extending from the handle portion;
a mirror part, which is detachably attached to an end of the neck portion, wherein
the attachment has a reflective surface on at least one surface to observe a surgical portion in an oral cavity, wherein
the neck portion is angled or curved in a side view of the retractor,
the neck portion has a connecting portion to which the mirror part is connected.

2. The dental retractor according to claim 1, wherein
the mirror part has reflective surfaces on both surfaces.

3. The dental retractor according to claim 1, wherein
the mirror part has a main body having a plate shape and a projection protruding from the main body, width of the projection being narrower than width of the main body, such that the projection can be inserted in to a hole formed at a surgical site to observe an inside the hole.

4. The dental retractor according to claim 1, wherein
the mirror part comprises a metallic material and has the reflective surfaces on both side.

5. The dental retractor according to claim 1, wherein
the mirror part is connected to the neck portion by a screw.

6. The dental retractor according to claim 1, wherein
the mirror part is connected to the neck portion by at least two screw.

7. The dental retractor according to claim 1, wherein
the connecting portion of the neck portion has at least one screw hole and the attachment is connected to the neck portion by at least one screw.

8. The dental retractor according to claim 6, further comprising a protector, wherein
the protector has a rectangular slit or hole, to which the attachment is slidably inserted, so that the protector fully or partially cover the mirror part.

9. A method for treating an oral cavity by the retractor of claim 1, the method comprises:
incising a skin tissue;
insert the retractor into an oral cavity;
observing an inside of the oral cavity by the mirror part, while holding the skin tissue that has been incised with the retractor open, so that a tooth or a tooth root can be observed.

10. The method according to claim 9, wherein the mirror part has serrations to provide support stability when in contact with a bone structure.

11. The dental retractor of claim 1, wherein the mirror part has a plate shape and a protruding part, and the mirror is narrower than the main body so that the mirror can be inserted into the hole from the incision at the surgical site allowing observation of the inside of the hole.

12. The method of claim 10, wherein the bone structure is a tooth.
